Europäisches Patentamt

(19) European Patent Office

Office européen des brevets

(11) Numéro de publication: **0 074 334**
**A1**

(12) **DEMANDE DE BREVET EUROPEEN**

(21) Numéro de dépôt: 82810353.1

(22) Date de dépôt: 26.08.82

(51) Int. Cl.³: **A 61 G 7/06**
**A 61 F 5/04**

(30) Priorité: 28.08.81 CH 5539/81

(43) Date de publication de la demande:
16.03.83 Bulletin 83/11

(84) Etats contractants désignés:
DE FR GB IT

(71) Demandeur: Gonthier, Claude
Ch. Magnette 13
CH-1350 Orbe(CH)

(72) Inventeur: Gonthier, Claude
Ch. Magnette 13
CH-1350 Orbe(CH)

(74) Mandataire: Seehof, Michel et al,
c/o AMMANN PATENTANWAELTE AG BERN
Schwarztorstrasse 31
CH-3001 Bern(CH)

(54) **Appareil pour le support et traitement de membres inférieures.**

(57) L'appareil de support est composé d'un cadre de base (1), extensible en longueur et en largeur, de deux barres d'élévation (3) extensible et liées par une articulation (2) au cadre de base et par une deuxième articulation supérieure (5) à un cadre de soutien (4) extensible en longueur et en largeur. Le cadre de soutien (4) est relié par deux barres de sécurité (9) au cadre de base (1). Les pièces des barres coulissant l'une dans l'autre sont fixées au moyen de vis imbus (7) et les deux articulations peuvent être arrêtées dans chaque position désirée. A chaque barre du cadre de soutien et des barres d'élévation est fixée une sangle (17).

Un tel appareil peut être adapté à des malades de grandeurs différentes, qu'il s'agisse d'adultes ou d'enfants et il peut être fixé dans toutes les positions désirées pour faciliter le bien-être, la guérison et le traitement de la jambe malade.

FIG.1

Croydon Printing Company Ltd.

<u>Appareil pour le support et traitement de membres</u>
<u>inférieures.</u>

La présente invention se rapporte à un appareil pour le
support et traitement de membres inférieures, cet appareil étant destiné à s'appuyer au pied du lit et pour
l'utilisation dans un hôpital.

Il existe un grand nombre de tels appareils, par exemple,
un appareil décrit dans le brevet suisse 435 552 et qui
est destiné à maintenir le pied d'un malade alité dans
sa position naturelle par rapport à la jambe et de permettre en même temps d'exécuter une certaine gymnastique
du pied. Cet appareil a une construction relativement
compliquée et il est difficile de l'adapter à différentes grandeurs de malades. Le brevet suisse 31 097 décrit
un support pour une jambe et permettant la fixation et
le traitement de cette dernière. Ce support comprend un
cadre avec une charnière pour permettre la fixation de
la jambe sous un certain angle, tandis que la jambe est
tenue par un filet et par plusieurs sangles. Cet appareil permet un certain changement de la position de la
jambe, mais il n'est pas prévu de s'adapter aux différentes longueurs et largeurs d'une jambe. D'autres appareils en utilisation dans des hôpitaux présentent les
mêmes défauts, c'est-à-dire une possibilité d'adaptation très restreinte.

C'est par contre l'objet de la présente invention de
mettre à disposition un appareil pour le support et le
traitement d'une jambe pouvant d'une part s'adapter à
toutes les grandeurs du malade, qu'il soit adulte ou enfant et pouvant être réglé dans toutes les positions nécessaires pour le traitement du membre malade et pour la
guérison de ce membre. Cet objet est atteint par un appareil décrit dans la partie descriptive de la première
revendication.

Dans ce qui suit, une forme de réalisation de l'invention sera décrite, à titre d'exemple, à l'aide du dessin annexé, dans lequel

la figure 1 montre en perspective un appareil selon l'invention, et

la figure 2 montre un détail de l'attachement d'une sangle.

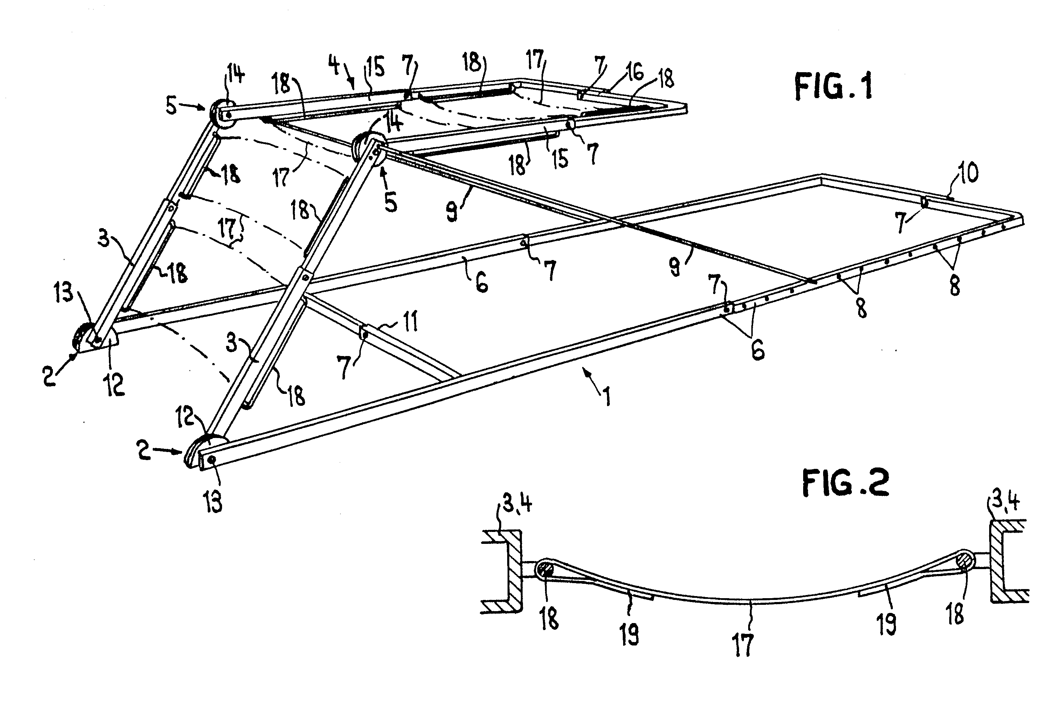

On reconnaît sur la figure 1 l'appareil comprenant un cadre de base 1 avec une première articulation 2, deux barres d'élévation 3 et un cadre de soutien 4, attachés avec deux articulations supérieures 5 aux deux barres d'élévation 3. Le cadre de base 1 se compose de deux barres longitudinales 6, ces barres étant extensibles, comprenant chacune deux pièces coulissant l'une dans l'autre et fixées par deux vis imbus 7. Les barres longitudinales comportent des trous 8 pour l'introduction d'une barre de sécurité 9 attachée d'une manière articulée au cadre de soutien 4. Le cadre de base 1 comprend en outre deux barres d'écartement 10 et 11, ces barres étant aussi extensibles, les deux pièces coulissant l'une dans l'autre et pouvant être fixées par une vis imbus 7. La première barre d'écartement 10 se trouve à l'extrémité côté pied de lit et la deuxième est fixée à une certaine distance de l'autre extrémité. A cette autre extrémité se trouvent les deux premières articulations de base 2, pouvant être réalisées de différentes façons. Une réalisation possible consiste en deux plaques semi-circulaires 12 avec une denture, ces plaques pouvant être serrées par une vis centrale 13. Il est clair qu'on pourrait aussi prendre une plaque avec des impressions dans une desquelles s'enfoncerait l'extrémité d'une vis imbus. L'une des deux plaques 12 est fixée à la barre longitudinale 6 et l'autre à une pièce coulissante de l'une des deux barres d'élévation extensibles. La deuxième pièce de la barre d'élévation 3, coulissant dans la première pièce, comporte une plaque circulaire 14 dentée de l'articulation supérieure 5. La deuxième plaque circulaire de l'articulation 5 est fixée à la barre longitudinale 15 du cadre de soutien 4, cette barre étant aussi en deux pièces coulissant l'une dans l'autre et pouvant être arrêtées par une vis imbus 7. Cette articulation est aussi serrée par une vis centrale et ainsi réglable dans une position angulaire désirée. Le cadre de soutien 4 comporte aussi une barre d'écartement 16, elle aussi composée de deux pièces coulissant l'une dans l'autre et pouvant être arrêtées par une vis imbus 7.

La jambe du malade repose sur quatre sangles 17, une sangle étant attachée à chaque pièce de la barre d'élévation et des barres longitudinales du cadre de soutien. Pour ce faire, chaque pièce de ces barres mentionnées comporte des supports 18, ces supports étant arrangés par rapport au support de la même barre de telle manière que ces dernières puissent coulisser l'une dans l'autre avec les sangles attachées. Une manière très favorable pour attacher les sangles est l'utilisation d'une fermeture à cramponner 19, connue sous la marque enregistrée "velcro". Une telle fermeture facilite énormément l'ouverture et le réglage de ces sangles.

Il ressort de la description de l'appareil que celui-ci offre de nombreuses possibilités d'adaptation, c'est-à-dire, qu'il peut être adapté d'une part à la longueur et à la largeur de la jambe du malade, qu'il s'agisse d'un adulte ou d'un enfant et d'autre part à une multitude de positions facilitant le bien-être, la guérison et le traitement du membre malade. Il est évident que du fait que la ou les sangles peuvent être enlevées aisément, le traitement, par exemple le changement de pansement ou l'examen de la partie intéressante de la jambe peut être exécuté de manière efficace et simple.

Cet appareil peut être utilisé pour un traitement à extension continue avec poids et parce qu'il est appuyé au pied du lit, il ne peut pas glisser et le malade ne peut pas appuyer sa jambe valide contre le pied du lit, ce qui arrive avec les appareils existants.

Les différentes barres coulissant l'une dans l'autre peuvent être réalisées de différentes manières, par exemple en utilisant des barres rectangulaires ou circulaires, dont l'une coulisse complètement dans l'autre ou en utilisant des barres rectangulaires avec des profils appropriés coulissant l'un dans l'autre. La fixation des pièces des barres extensibles ne doit pas absolument se faire par des vis imbus, mais il est souhaitable que le malade ne puisse pas déserrer lui-même les vis en question. Comme décrit auparavant, les articulations peuvent être réalisées de différentes manières, mais il est évident qu'il est nécessaire de pouvoir les fixer dans chaque position désirée. La barre de sécurité 9 ne doit pas être obligatoirement fixée près de la deuxième articulation comme indiqué en figure 1, mais elle peut être fixée auprès de la barre d'écartement 16 du cadre de soutien. Les sangles 17 peuvent être attachées par deux fermetures 19 ou alors en prenant soin que la sangle ne puisse pas sortir du support d'un côté, par exemple à l'aide d'une barre introduite dans l'extrémité de la sangle et en n'utilisant qu'une fermeture 19 à l'autre extrémité de cette sangle. Il est évident qu'il est possible d'utiliser aussi tout autre genre de sangle attachée d'une manière conventionnelle par des courroies ou des lacets. Comme matériaux pour la construction de cet appareil sont utilisés les matériaux courants pour ce genre d'appareil médical, par exemple du laiton chromé. Elle peut être prévue en deux pièces coulissant l'une dans l'autre.

Revendications

1. Appareil pour le support et traitement de membres inférieures, caractérisé par un cadre de base (1) extensible en longueur et en largeur, deux barres d'élévation (3) extensibles en longueur, chacune connectée par une première articulation (2) réglable angu- lairement audit cadre de base et un cadre de soutien (4) extensible en longueur et en largeur, connecté par des articulations supérieures (5) réglables angu- lairement audites barres d'élévation.

2. Appareil selon la revendication 1, caractérisé par deux barres de sécurité (9) reliées d'une manière mobile audit cadre de soutien (4) et s'engageant dans des trous (8) pratiqués dans les barres longitudinales (6) du cadre de base (1).

3. Appareil selon la revendication 1, caractérisé en ce que le cadre de base (1) se compose de deux barres longitudinales (6) et de deux barres d'écartement (10, 11) et le cadre de soutien (4) de deux barres longitudinales (15) et d'une barre d'écartement (16), chacune desdites barres étant composée de deux pièces coulissant l'un dans l'autre et pouvant être fixée dans chaque position désirée.

4. Appareil selon la revendication 3, caractérisé en ce que les pièces coulissantes des barres peuvent être fixées par des vis imbus (7).

5. Appareil selon l'une quelconque des revendications 1 à 4, caractérisé en ce que chacune des pièces des barres longitudinales du cadre de soutient et des barres d'élévation comporte un support de sangles (18), ces supports étant arrangés de telle manière que les pièces des barres peuvent être glissées l'une

0074334

dans l'autre.

6. Appareil selon la revendication 5, caractérisé en ce que chaque paire desdites pièces de barres comporte une sangle (17) fixée de manière détachable.

7. Appareil selon la revendication 6, caractérisé en ce que les sangles (17) sont fixées par des fermetures connues sous la marque déposée "velcro".

8. Appareil selon la revendication 2, caractérisé en ce que chaque barre de sécurité se compose de deux pièces coulissant l'une dans l'autre et pouvant être fixée.

**FIG.1**

**FIG.2**

0074334

**0074334**
Numéro de la demande

Office européen
des brevets

**RAPPORT DE RECHERCHE EUROPEENNE**

EP 82 81 0353.1

| Catégorie | Citation du document avec indication, en cas de besoin, des parties pertinentes | Revendication concernée | CLASSEMENT DE LA DEMANDE (Int. Cl. ³) |
|---|---|---|---|
| | **DOCUMENTS CONSIDERES COMME PERTINENTS** | | |
| Y | DD - A - 43 361 (E. KAMINSKI) | 1-3 | A 61 G 7/06 |
| | * document complet * | 5,6,8 | A 61 F 5/04 |
| | --- | | |
| Y | DE - A - 2 038 487 (W.-E. TWARDZIK et al.) | 1,3,8 | |
| | * revendications 1, 2, 8 * | | |
| | --- | | |
| Y | DE - A - 1 791 043 (E. WALLSTEINER) | 2 | DOMAINES TECHNIQUES RECHERCHES (Int. Cl. ³) |
| | * revendications 1, 2 ; fig. 1 * | | |
| | --- | | |
| Y | US - A - 3 848 589 (G.C. THRONER) | 1,3,5,6 | A 47 C 20/00 |
| A | * résumé ; colonne 3, lignes 37 à | 4 | A 61 F 5/00 |
| | 40, 50 à 57, 63, 64 * | | A 61 G 7/06 |
| | --- | | |
| A | US - A - 3 640 273 (T.D. RAY) | 7 | |
| | * colonne 2, lignes 69 à 75 ; colonne 3, lignes 10, 11 * | | |
| | --- | | |
| A | DE - C - 317 966 (A. KAUFMANN) | | |
| | --- | | CATEGORIE DES DOCUMENTS CITES |
| P,A | US - A - 4 336 796 (E.T. ANDREWS et al.) | 1,6,7 | X: particulièrement pertinent à lui seul<br>Y: particulièrement pertinent en combinaison avec un autre document de la même catégorie<br>A: arrière-plan technologique<br>O: divulgation non-écrite<br>P: document intercalaire<br>T: théorie ou principe à la base de l'invention<br>E: document de brevet antérieur, mais publié à la date de dépôt ou après cette date<br>D: cité dans la demande<br>L: cité pour d'autres raisons |
| | * colonne 2, lignes 30 à 35 ; colonne 3, lignes 56 à 59 ; fig. 2, 6 * | | |
| | ---- | | |
| | | | &: membre de la même famille, document correspondant |

X Le présent rapport de recherche a été établi pour toutes les revendications

| Lieu de la recherche | Date d'achèvement de la recherche | Examinateur |
|---|---|---|
| Berlin | 11-11-1982 | CLOT |

OEB Form 1503.1   06.78